Europäisches Patentamt

European Patent Office

Office européen des brevets

(11) Numéro de publication: **0 407 257 A2**

# (12) DEMANDE DE BREVET EUROPEEN

(21) Numéro de dépôt: 90401742.3

(22) Date de dépôt: 20.06.90

(51) Int. Cl.5: **B01J 13/02**

(30) Priorité: 30.06.89 FR 8908777

(43) Date de publication de la demande:
09.01.91 Bulletin 91/02

(84) Etats contractants désignés:
AT BE CH DE ES GB IT LI LU NL SE

(71) Demandeur: HUTCHINSON S.A.
2 rue Balzac
F-75008 Paris(FR)

(72) Inventeur: Pense, Anne-Marie
3, Villa Bac
F-94200 Ivry-Sur-Seine(FR)
Inventeur: Vauthier, Christine
1, Place des Prés Bouchards
F-91370 Verrières-le-Buisson(FR)
Inventeur: Benoit, Jean-Pierre
5, Avenue Emile Savigner
F-49240 Avrille(FR)

(74) Mandataire: Ores, Irène et al
CABINET ORES 6, Avenue de Messine
F-75008 Paris(FR)

(54) Microcapsules contenant des substances amphiphiles hydrosolubles.

(57) La présente invention est relative à des microcapsules contenant des substances amphiphiles hydrosolubles et à leur procédé d'obtention par polymérisation ou polycondensation interfaciale, ainsi qu'à leurs applications.

EP 0 407 257 A2

## MICROCAPSULES CONTENANT DES SUBSTANCES AMPHIPHILES HYDROSOLUBLES

La présente invention est relative à des microcapsules contenant des substances amphiphiles hydrosolubles et à un procédé permettant de les obtenir.

Les microcapsules sont des particules de forme sphérique, dont la taille s'échelonne entre 1 et 1250 $\mu$m, constituées d'un matériau support, contenant la substance encapsulée. Suivant la structure du matériau de support, on distingue deux types de microcapsules :

- les microcapsules de type réservoir dans lesquelles le matériau support est une enveloppe solide, d'épaisseur variable contenant la substance à encapsuler,
- les microcapsules de type matriciel appelées également microsphères dans lesquelles le matériau support est un réseau continu, dans lequel est dispersée la substance à encapsuler.

Au sens de la présente invention, le terme microcapsule englobe aussi bien les microcapsules de type matriciel que les microcapsules de type réservoir.

Le matériau support limite les échanges entre la substance encapsulée et le milieu extérieur, ce qui a pour effet de protéger ladite substance, et de permettre son incorporation à des produits dans lesquels il n'aurait pas été possible de l'incorporer à l'état libre. En outre, l'utilisation de substances microencapsulées permet de contrôler la libération de celles-ci, en en différant l'instant - la substance est alors libérée par destruction de la paroi de la microcapsule - ou en l'étalant dans le temps - la substance est libérée par diffusion progressive à travers la paroi -.

De nombreuses substances peuvent être encapsulées : il peut s'agir de produits chimiques tels que des médicaments ou des pesticides, ou bien de macromolécules telles que des enzymes, et également de cellules vivantes.

Les microcapsules sont utilisées dans de nombreux domaines tels que la pharmacie, la bio-industrie, les cosmétiques, l'agroalimentaire, l'industrie papetière, etc...

Des exemples d'applications de la microencapsulation sont détaillés dans l'article de C. DUBERNET et J.P. BENOIT paru dans "l'Actualité Chimique" ; (Décembre 1986). .

L'un des procédés utilisé pour encapsuler des substances liquides est la microencapsulation par polycondensation interfaciale. La polycondensation interfaciale est une réaction de polymérisation qui se produit à l'interface de deux liquides non miscibles, dont au moins l'un des deux contient un réactif polyfonctionnel approprié. Dans le cas où cette réaction s'effectue sans formation de produits secondaires, elle est également appelée polymérisation interfaciale. Cette réaction peut être réalisée dans une émulsion, à l'interface entre les deux phases. Dans ce cas, elle aboutit à la formation de microcapsules réservoir, si les oligomères formés au début de la réaction sont insolubles dans la phase dispersée, et de microcapsules matricielles si lesdits oligomères sont solubles dans la phase dispersée.

Ce procédé qui a été décrit, par exemple, dans le Brevet français 1 427 085 et, plus récemment dans la publication de R. ARSHADY, [J. MICROENCAPSULATION 1989, Vol. 6 N°1], ainsi que dans la publication de C. DUBERNET et J. BENOIT citée plus haut, permet de préparer des capsules dont la paroi est formée de polyamide, de polyuréthane, de polyurée, de polyester, etc...

Le Brevet Belge N° 796 746 décrit également un procédé d'obtention, à partir d'une émulsion huile/eau, de microcapsules dont la paroi est formée de polyurée, par polymérisation d'un isocyanate, présent dans la phase organique. Le Brevet Français N° 2 548 046 décrit un procédé basé sur le même principe, pour l'encapsulation de substances hydrosolubles, à partir d'une émulsion eau/huile.

Ces procédés conviennent à l'encapsulation de nombreuses substances hydrophiles ou lipophiles. Toutefois, ils ne permettent pas l'encapsulation de substances amphiphiles hydrosolubles telles que, par exemple, les ammoniums quaternaires.

Or, les ammoniums quaternaires sont utilisés entre autres pour leurs propriétés antiseptiques. Par exemple, le chlorure de benzalkonium est utilisé en applications locales, comme bactéricide et fongicide. Son action sur le virus HIV a également été démontrée. Cependant, les ammoniums quaternaires sont incompatibles avec de nombreuses substances, notamment anioniques telles que les savons. En outre, ils sont communément utilisés en solution, ce qui ne permet pas de les associer commodément à un support solide. Il serait donc particulièrement avantageux de les encapsuler.

Toutefois, cette encapsulation pose des problèmes spécifiques qui n'avaient pas été résolus jusqu'à présent.

Les ammoniums quaternaires étant hydrosolubles, il est nécessaire, pour les encapsuler, d'obtenir une émulsion stable du type eau dans l'huile. Or, les ammoniums quaternaires et de façon plus générale, les substances amphiphiles hydrosolubles favorisent les émulsions du type huile dans l'eau, et déstabilisent les émulsions du type eau/huile. En outre, il est connu que les ammoniums quaternaires interfèrent avec la

2

réaction de polymérisation interfaciale. C'est ainsi que, par exemple Mc GINITY et al. [J. Pharm., 70 , 372 (1981)] et ARSHADY [J. Microencapsulation, 6 , 18-23, 1989], considérent qu'il n'est pas possible d'obtenir des microcapsules en présence d'ammonium quaternaire.

Or, les Inventeurs ont maintenant mis au point un procédé qui, de façon surprenante, permet d'obtenir des microcapsules contenant des substances amphiphiles hydrosolubles, y compris des ammoniums quaternaires.

La présente invention a en conséquence pour objet des microcapsules, caractérisées en ce qu'elles contiennent au moins une substance amphiphile hydrosoluble.

Selon un mode de réalisation préféré de ces microcapsules, la substance amphiphile hydrosoluble est une substance cationique, en particulier un ammonium quaternaire.

Selon une modalité particulièrement avantageuse de ce mode de mise en oeuvre, la substance amphiphile hydrosoluble est le chlorure de benzalkonium.

Selon un autre mode de réalisation préféré de ces microcapsules la substance amphiphile hydrosoluble est une substance non ionique, en particulier un dérivé hydrosoluble de polyoxyéthylène.

Selon une modalité particulièrement avantageuse de ce mode de réalisation la substance amphiphile hydrosoluble est un nonoxynol dont le nombre moyen d'unités d'oxyde d'éthylène par molécule est supérieur à 6.

Selon une modalité particulièrement avantageuse de ce mode de réalisation la substance amphiphile hydrosoluble est un octoxynol dont le nombre moyen d'unités d'oxyde d'éthylène par molécule est supérieur à 6.

Selon un autre mode de réalisation préféré de la présente invention, la substance amphiphile hydrosoluble est une substance anionique, en particulier un alkyl sulfate de sodium.

Selon une modalité particulièrement avantageuse de ce mode de réalisation la substance amphiphile hydrosoluble est le lauryl sulfate de sodium.

Selon un autre mode de réalisation préféré de la présente invention, la paroi des microcapsules est constituée essentiellement de polyurée. Elle peut également être constituée de tout autre polymère résultant d'une réaction de polymérisation ou de polycondensation interfaciale, tels que par exemple les polyesters polyamides ou polyuréthanes.

La présente invention a en outre pour objet un procédé d'encapsulation de substances amphiphiles hydrosolubles, par polycondensation ou par polymérisation interfaciale, qui comprend une première étape de réalisation d'une émulsion d'une phase aqueuse contenant la substance à encapsuler dans une phase organique, et une deuxième étape de formation des microcapsules par polymérisation d'un ou plusieurs réactifs polyfonctionnels, lequel procédé est caractérisé en ce que la phase aqueuse contenant la substance amphiphile à encapsuler est émulsionnée, en présence d'un agent émulsifiant approprié, dans une phase organique contenant au moins un composant pris dans le groupe constitué par les hydrocarbures aliphatiques, alicycliques, ou aromatiques, halogénés ou non, et les triglycérides.

Selon un mode de mise en oeuvre préféré du procédé conforme à la présente invention, la HLB (balance hydrophile lipophile) de l'agent émulsifiant est inférieure à 7. Une HLB inférieure à 7 peut être obtenue en utilisant comme agent émulsifiant soit un tensio-actif, soit plusieurs tensio-actifs éventuellement associés à des agents abaissant la HLB, tels que des sels d'acide carboxyliques, comme par exemple l'acétate ou le formiate de sodium ou de lithium.

Selon une modalité avantageuse de ce mode de mise en oeuvre, l'agent émulsifiant est un copolymère de type polyester non ionique modifié, de HLB compris entre 5 et 7.

Selon une autre modalité avantageuse de ce mode de mise en oeuvre, l'agent émulsifiant est constitué par un mélange, en solution dans la phase organique, d'un polysiloxane (par exemple le polysiloxane 3225C) et d'un ester de sorbitan (par exemple le Span 85).

Selon encore une autre modalité avantageuse de ce mode de mise en oeuvre, l'agent émulsifiant est constitué par une association de Polysorbate 80, en solution dans la phase aqueuse, et d'un ester de sorbitan (par exemple le Span 85), en solution dans la phase organique.

Selon un autre mode de mise en oeuvre préféré du procédé conforme à la présente invention, la phase organique contient un mélange de triglycérides.

Selon un autre mode de mise en oeuvre du procédé conforme à la présente invention, le ou les triglycérides contenus dans la phase organique est/sont un/des triglycéride(s) d'acides gras en $C_8$ à $C_{18}$.

Selon encore un autre mode de mise en oeuvre du procédé conforme à la présente invention, la phase organique est constituée par un mélange chloroforme/cyclohexane.

Selon encore un autre mode de mise en oeuvre du procédé conforme à la présente invention, la phase organique contient du xylène ou du toluène.

Le procédé d'encapsulation conforme à la présente invention peut également être mis en oeuvre dans une phase organique constituée exclusivement de triglycérides.

Les Inventeurs ont en outre constaté qu'en faisant varier les proportions respectives d'hydrocarbures et de triglycérides, il est possible d'obtenir des capsules de résistance variable, qui peuvent donc être adaptées à des usages divers.

Selon un mode de mise en oeuvre préféré du procédé conforme à la présente invention, la phase organique est un mélange hydrocarbures/triglycérides qui contient entre 10 et 50%, en poids, d'hydrocarbures.

La plupart des réactifs polyfonctionnels habituellement utilisés dans les procédés de polymérisation interfaciale peuvent être utilisés dans le procédé selon la présente invention, pour la formation des parois des microcapsules.

Par exemple les diisocyanates peuvent être utilisés pour obtenir des microcapsules dont la paroi est essentiellement constituée de polyurée.

Les microcapsules conformes à la présente invention peuvent de façon générale être utilisées dans toutes les applications des microcapsules. De façon non limitative elles peuvent être incorporées à des liquides, des pâtes, des crèmes, des savons, des peintures et vernis, ou bien des matières solides telles que des papiers, des textiles, des éponges, des matériaux à base de polymères, en particulier d'élastomères.

La présente invention sera mieux comprise à l'aide du complément de description qui va suivre, qui se réfère à des exemples de préparation de microcapsules suivant le procédé conforme à la présente invention.

Il doit être bien entendu, toutefois, que ces exemples dont donnés uniquement à titre d'illustration de l'objet de l'invention, dont ils ne constituent en aucune manière une limitation.

## I - PREPARATION DES MICROCAPSULES CONFORMES A LA PRESENTE INVENTION

### EXEMPLE 1

#### A) PHASE ORGANIQUE

15g de diphénylméthane diisocyanate et 6g de A 60 sont dissous dans 60g de xylène, 140g de MYGLIOL 812 sont ensuite ajoutés. (Le A 60 (Laboratoires ICI) est un tensio-actif non ionique ; il s'agit d'un polyester modifié. Le MYGLIOL 812 est un mélange de triglycérides d'acides gras saturés et fractionnés de coco, à longueur de chaîne $C_8$-$C_{10}$).

#### B) PHASE AQUEUSE

0,3g de chlorure de benzalkonium sont dissous dans 30ml d'eau.

La phase aqueuse est émulsionnée dans la phase organique au moyen d'un microvortex à forte puissance pendant 1 à 2 minutes. L'émulsion ainsi préparée est placée sous agitation douce (pale, 300tpm) pendant la durée de la réaction (8 à 10h). Les microcapsules obtenues sont récupérées après décantation. Elles sont ensuite lavées au cyclohexane puis séchées à température ambiante. 45g de microcapsules d'une taille comprise entre 30 et 50 $\mu$m sont ainsi obtenues. La quantité de chlorure de benzalkonium encapsulé est déterminée par HPLC selon la technique suivante : une quantité déterminée de microcapsules est dissoute dans 5ml de diméthyl formamide. 5ml d'eau sont ajoutés à cette solution pour repréciter le polymère. La suspension résultante subit une ultracentrifugation à 35.000 tpm pendant lh30. Le surnageant après passage sur filtre de 0,2 $\mu$m est dosé par HPLC suivant les modalités opératoires suivantes : 30 $\mu$l de l'échantillon sont analysés par passage sur une colonne NOVAPACK CN (WATERS) avec une phase mobile composée pour 60% d'acétonitrile et pour 40% d'un tampon acétate, à un débit de 2ml/mn. La détection du pic correspondant au chlorure de benzalkonium est réalisée à 254 nm. Le rendement d'encapsulation du chlorure de benzalkonium, déterminé suivant cette méthode est de 40%.

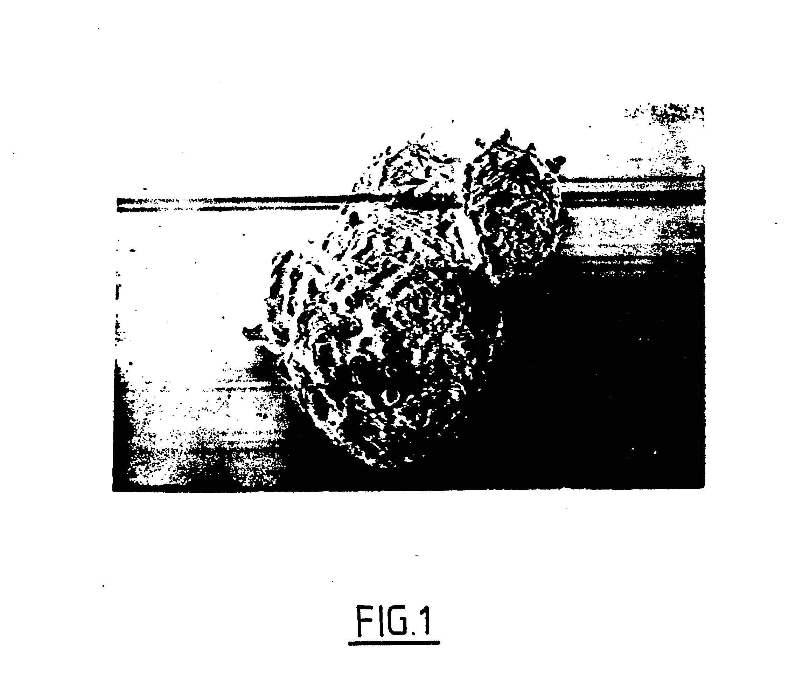

La figure 1 montre l'aspect des capsules obtenues, observées au microscope électronique à balayage.

### EXEMPLE 2

4

## A) PHASE ORGANIQUE

15g de diphénylméthane diisocyanate et 12g de A 60 sont dissous dans 40g de xylène auquel sont ajoutés ensuite 240g de MYGLIOL 812.

## B) PHASE AQUEUSE

3g de chlorure de benzalkonium sont dissous dans 30ml d'eau. La suite de la procédure est identique à celle décrite dans l'exemple 1.

40g de microcapsules d'une taille comprise entre 50 et 70 μm sont récupérées. Le rendement d'encapsulation est de 60% soit 46mg de chlorure de benzalkonium pour 1g de capsules.

## EXEMPLE 3 :

### A) PHASE ORGANIQUE

7 g de diphénylméthane diisocyanate et 4 g de A 60 sont dissous dans 30 g de toluène. 170 g de MIGLYOL 840 sont ajoutés à la solution.

### B) PHASE AQUEUSE

0,3 g de chlorure de benzalkonium sont dissous dans 30ml d'eau.

La suite de la procédure est identique à celle décrite dans l'Exemple 1. 13 g de microcapsules sont récupérés.

## EXEMPLE 4 :

### A) PHASE ORGANIQUE

15 g de diphénylméthane diisocyanate et 7,5 g de Span 85 sont dissous dans 30 g de xylène, auxquels sont ajoutés 170 g de MYGLIOL 812.

### B) PHASE AQUEUSE

2,5 g de Tween 80 et 0,3 g de chlorure de benzalkonium sont dissous dans 30ml d'eau. L'ensemble est émulsionné dans le mélange précédent.

La suite de la procédure est identique à celle décrite dans l'Exemple 1. 18 g de microcapsules sont récupérées.

## EXEMPLE 5 :

### A) PHASE ORGANIQUE

10 g de diphénylméthane diisocyanate et 5 g de A 60 sont dissous dans 40 g de xylène, auxquels sont ajoutés 160 g de MIGLYOL 812.

### B) PHASE AQUEUSE

1 g de laurylsulfate de sodium sont dissous dans 30ml d'eau.

La suite de la procédure est identique à celle décrite dans l'Exemple 1. Les microcapsules ont une taille moyenne de 10 µm.

## EXEMPLE 6 :

### A) PHASE ORGANIQUE

15 g de diphénylméthane diisocyanate et 7 g de A 60 sont dissous dans 200 g de xylène.

### B) PHASE AQUEUSE

3 g de Triton X 100 sont dissous dans 30 g d'eau.

La suite de la procédure est identique à celle décrite dans l'Exemple 1. La taille des microcapsules obtenues varie entre 10 et 50 µm.

## EXEMPLE 7 :

### A) PHASE ORGANIQUE

15 g de diphénylméthane diisocyanate, 5 g de polysiloxane 3225 C (DOW CORNING) et 5 g de Span 85 sont dissous dans 200 g de xylène.

### B) PHASE AQUEUSE

3 g de Nonoxynol 9 sont dissous dans 30ml d'eau.

La suite de la procédure est identique à celle décrite dans l'Exemple 1.

## EXEMPLE 8 :

### A) PHASE ORGANIQUE

10 g de diphénylméthane diisocyanate et 5 g de A 60 sont dissous dans 200 g d'un mélange cyclohéxane/chloroforme dans un rapport 4/1.

### B) PHASE AQUEUSE

1 g de chlorure de benzalkonium est dissous dans 30 ml d'eau.

La suite de la procédure est identique à celle décrite à l'Exemple 1.

## II - INFLUENCE DE LA PHASE ORGANIQUE SUR LES PROPRIETES DES MICROCAPSULES

### A) INFLUENCE SUR LES PROPRIETES MECANIQUES

Le tableau I ci-après montre l'influence de la composition de la phase organique sur les propriétés de résistance mécanique des microcapsules préparées à partir d'une solution à 1% de chlorure de benzalkonium.

EP 0 407 257 A2

TABLEAU I

| PHASE ORGANIQUE | RESISTANCE |
|---|---|
| XYLENE 100% | 0 |
| XYLENE 80% MYGLIOL 20% | 0 |
| XYLENE 60% MYGLIOL 40% | 0 |
| XYLENE 50% MYGLIOL 50% | 0 + |
| XYLENE 40% MYGLIOL 60% | + |
| XYLENE 30% MYGLIOL 70% | + + + |
| XYLENE 15% MYGLIOL 85% | + + + |
| MYGLIOL 100% | + + |

La légende de ce tableau est la suivante :

| Microcapsules très fragiles | O |
|---|---|
| Microcapsules fragiles | + |
| Microcapsules résistantes | + + |
| Microcapsules très resistantes | + + + |

B) INFLUENCE SUR LA TENEUR EN EAU DES MICROCAPSULES ET SON EVOLUTION AU COURS DU TEMPS

La teneur en eau est évaluée par mesure de la perte de poids à la dessication par chauffage à l'étuve pendant 1 heure à 105° C.

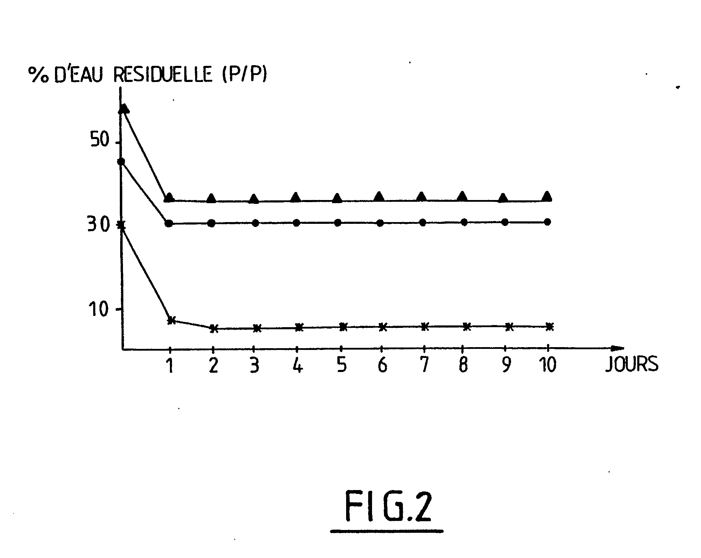

La figure 2 représente le pourcentage d'eau résiduelle dans les microcapsules en fonction du temps de conservation pour des microcapsules préparées à partir d'une solution à 1% de chlorure de benzalkonium, dans 3 phases lipidiques de composition différente

▲——▲    XYLENE  15% MYGLIOL  85%

●——●    XYLENE  40% MYGLIOL  60%

✱——✱    MYGLIOL  100%

Ainsi que cela ressort de ce qui précède, l'invention ne se limite nullement à ceux de ces modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

**Revendications**

1°) Microcapsules, caractérisées en ce qu'elles contiennent au moins une substance amphiphile hydrosoluble. `

2°) Microcapsules selon la Revendication 1, caractérisées en ce que la substance amphiphile hydrosoluble est une substance cationique, en particulier un ammonium quaternaire.

3°) Microcapsules selon la Revendication 2, caractérisées en ce que la substance amphiphile hydrosoluble est le chlorure de benzalkonium.

4°) Microcapsules selon la Revendication 1, caractérisées en ce que la substance amphiphile hydrosoluble est une substance non ionique, en particulier un dérivé hydrosoluble de polyoxyéthylène.

5°) Microcapsules selon la Revendication 4, caractérisées en ce que là substance amphiphile hydrosoluble est un nonoxynol dont le nombre moyen d'unités d'oxyde d'éthylène par molécule est supérieur à 6.

6°) Microcapsules selon la Revendication 4, caractérisées en ce que la substance amphiphile hydrosoluble est un octoxynol dont le nombre moyen d'unités d'oxyde d'éthylène par molécule est supérieur à 6.

7°) Microcapsules selon la Revendication 1, caractérisées en ce que la substance amphiphile hydrosoluble est une substance anionique, en particulier un alkyl sulfate de sodium.

8°) Microcapsules selon la Revendication 7, caractérisées en ce que la substance amphiphile hydrosoluble est le lauryl sulfate de sodium.

9°) Microcapsules selon la Revendication 1, caractérisées en ce que la paroi des microcapsules est constituée essentiellement de polyurée.

10°) Procédé d'encapsulation de substances amphiphiles hydrosolubles, par polycondensation ou par polymérisation interfaciale, qui comprend une première étape de réalisation d'une émulsion d'une phase aqueuse contenant la substance à encapsuler dans une phase organique, et une deuxième étape de formation des microcapsules par polymérisation d'un ou plusieurs réactifs polyfonctionnels, lequel procédé est caractérisé en ce que la phase aqueuse contenant la substance amphiphile à encapsuler est émulsionnée, en présence d'un agent émulsifiant approprié, dans une phase organique contenant au moins un composant pris dans le groupe constitué par, les hydrocarbures aliphatiques, alicycliques, ou aromatiques, halogénés ou non, et les triglycérides.

11°) Procédé selon la Revendication 10, caractérisé en ce que la HLB (balance hydrophile lipophile) de l'agent émulsifiant est inférieure à 7.

12°) Procédé selon la Revendication 10, caractérisé en ce qu'on utilise comme agent émulsifiant par au moins un tensio-actif, éventuellement associé à un agent abaissant la HLB choisi parmi le groupe comprenant les tensio-actifs et les sels en particulier les sels de métaux basiques d'acides carboxyliques.

13°) Procédé selon l'une quelconque des Revendications 10 ou 11, caractérisé en ce que l'agent émulsifiant est un copolymère de type polyester non ionique modifié, de HLB compris entre 5 et 7.

14°) Procédé selon l'une quelconque des Revendications 10 ou 11, caractérisé en ce que l'agent émulsifiant est constitué par un mélange, en solution dans la phase organique, de polysiloxane et d'un ester de sorbitan.

15°) Procédé selon l'une quelconque des Revendications 10 ou 11, caractérisé en ce que l'agent émulsifiant est constitué par une association de polysorbate 80, en solution dans la phase aqueuse, et d'un ester de sorbitan, en solution dans la phase organique.

16°) Procédé selon la Revendication 10, caractérisé en ce que la phase organique contient un mélange de triglycérides.

17°) Procédé selon la Revendication 10, caractérisé en ce que le ou les triglycérides contenus dans la phase organique est/sont un/des triglycéride(s) d'acides gras en $C_8$ à $C_{18}$.

18°) Procédé selon la Revendication 10, caractérisé en ce que la phase organique est constituée par un mélange chloroforme/cyclohexane.

19°) Procédé selon la Revendication 10, caractérisé en ce que la phase organique contient du xylène ou du toluène.

20°) Procédé selon la Revendication 10, caractérisé en ce que la phase organique est un mélange hydrocarbures/triglycérides qui contient entre 10 et 50%, en poids, d'hydrocarbures.

21°) Procédé selon la Revendication 10, caractérisé en ce que, dans l'étape de formation des microcapsules, le réactif polyfonctionnel utilisé est un diisocyanate.

22°) Produits liquides ou semi-solides, caractérisés en ce qu'ils contiennent des microcapsules selon l'une quelconque des Revendications 1 à 9.

23°) Papiers ou textiles, caractérisés en ce qu'ils contiennent des microcapsules selon l'une quelconque des Revendications 1 à 9.

24°) Produits à base de polymères, en particulier d'élastomères, caractérisés en ce qu'ils contiennent des microcapsules selon l'une quelconque des Revendications 1 à 9.

25°) Savons, caractérisés en ce qu'ils contiennent des microcapsules selon l'une quelconque des Revendications 1 à 9.

FIG.1

% D'EAU RESIDUELLE (P/P)

FIG.2

EP 0 407 257 A2